Europäisches Patentamt

European Patent Office    (11) Publication number: **0 112 155**

Office européen des brevets    **A2**

(12) **EUROPEAN PATENT APPLICATION**

(21) Application number: 83307581.5    (51) Int. Cl.³: **C 12 M 3/02**

(22) Date of filing: 13.12.83

(30) Priority: 15.12.82 US 450056

(43) Date of publication of application:
27.06.84 Bulletin 84/26

(84) Designated Contracting States:
CH DE FR GB IT LI

(71) Applicant: BIO-RESPONSE INC.
550 Ridgefield Road
Wilton Connecticut 06897(US)

(72) Inventor: Rose, Sam
2701 Claremont Boulevard
Berkeley California 94705(US)

(74) Representative: Marsh, Roy David et al,
Brewer & Son 5-9, Quality Court Chancery Lane
London WC2A 1HT(GB)

(54) A method and system for culturing and treating substances disposed in a flowing culture fluid.

(57) The disclosure relates to a method and system utilizing double flows for simultaneously culturing cells suspended in a flow of culture fluid (11) extending across one surface of a membrane (14) and removing waste products from the cell-containing fluids through the membrane from the opposite surface thereof. The disclosure further relates to a method and system for culturing cells suspended in a flow of culture fluid extending across one side of a membrane by supplying nutrients through the membrane by osmotic diffusion. There is also disclosed a method and system for utilizing double flows for simultaneously culturing, aerating and otherwise treating cells suspended in a flow of culture fluid extending across one side of a membrane.

Fig. 1.

A METHOD AND SYSTEM FOR CULTURING AND TREATING
SUBSTANCES DISPOSED IN A FLOWING CULTURE FLUID

BACKGROUND OF THE INVENTION

1.  Field of the Invention

This invention relates to methods and systems for culturing and treating mammalian cells and parasitic organisms (organisms which can only exist in complement with a mammalian cell or in a mammalian cell environment) suspended in a culture fluid.  More particularly, this invention relates to methods and systems for culturing and treating cells and organisms by means of the separate and independent flow of a culture fluid containing cells or parasitic organisms across one side of a membrane and the separate and independent flow of a treating fluid across the other side of the membrane.  Still more particularly the invention relates to methods and systems useable in culturing and treating cells and parasitic organisms suspended in fluid by sequential use of a series of different membranes supported within a leakproof housing in parallel relationship to one another.  The housing forms a series of separate compartments for the membranes.  A fluid containing cells or parasitic organisms may be independently flowed through a series of the separate compartments while other fluids or gases may be independently flowed through separate compartments which are adjacent to the compartments through which the cell containing fluid is being flowed. In accordance with the invention a double flow system is achieved with a flow of culture fluid containing cells or parasitic organisms on one side of a membrane and a flow of treating fluid on the other side of the membrane.

2.  Description of the Prior Art

The culturing of cells has conventionally been carried out in either batch processes or flow processes.

In batch processes, cells suspended in culture fluid are subjected to culturing in vessels such as tanks. Stirring of the culture fluid may be used to facilitate washing of the cells and the delivery of nutrients to them. Certifuges are used to enable the cells to be held along an outer wall of the vessel by certifugel force as washing cycles or changes of nutrients are carried out. A reduction in certifugel force enables the cells to disperse within the fluid, whether it be culture, nutrient or washing fluid. Within the vessel, the cells can be aerated by bubbling gas thereinto.

In flow processes, the cells which are suspended in culture fluid are circulated about a closed loop or path by pumping. At various stages along the loop the cells are subjected to the delivery of nutrients to the culture fluid and perhaps aeration of the culture fluid. Filters can be employed to remove waste products from the culture fluid.

Whether a batch process or a flow process is used, the cells are subjected to continuous manipulation or agitation in carrying out the various cycles of washing,

-3-

addition of nutrients, removal of waste products, and aeration. Such manipulation and agitation can be deleterious or even injurious to the condition of the cells. A typical example of a method wherein living cells are subjected to such a continuous manipulation in their culturing and other treatment is disclosed in U.S. Patent No. 3,964,467, issued June 22, 1976 to Sam Rose for "Method and Apparatus for Augmentation of Production of Anti-Bodies in Animals and Humans and the Collection Thereof". The disclosure of this patent is incorporated herein by reference.

It is also known in the art of filters to utilize membranes to remove a filtrate from one side of a membrane over which a fluid containing liquid and particles or solids is flowing. Such filters employ a single semi-permeable membrane across the surface of which is established a flow of fluid. Such filter systems employ a plate disposed adjacent one surface the semi-permeable membrane for supporting the membrane. The support plate is provided with grooves, openings or indentations which trap and collect filtrates diffusing through the membrane and delivers them to a common disposal outlet. Typical examples of the use of semi-permeable membranes for filtering only are disclosed in the Encyclopedia of Chemical Technology, 3d Edition, Vol. 15, Membrane Technology, pp. 92-111 and in The Way Things Work, 1st Edition, Vol. 2, Artificial Kidney, pp. 418-25, the disclosures of which are incorporated herein by reference.

In the prior art of dialysis it is known to flow a substance on one side of a membrane in order to diffuse unwanted waste materials from the substance and through the membrane. In a form of dialysis called hemodialysis, blood is flowed across one surface of a membrane while a liquid such as water is disposed on the opposite surface of the membrane. Due to the differences in concentration of waste materials in the blood fluid and the liquid, diffusion takes place and enables the waste material products to be extracted from the blood. In hemodialysis there is no culture of blood cells. Typical examples of the use of membranes used in hemodialysis filters are disclosed in the Encyclopedia of Chemical Technology, 3d Edition, Vol. 7, Dialysis, pp. 564-579, the disclosure of which is incorporated herein by reference.

## SUMMARY OF THE INVENTION

It is an object of the invention to provide a method and system for culturing cells suspended in culture fluid as the fluid flows across a membrane.

Another object of the invention is to provide a method and systems for removing waste products from a flow of cells in culture fluid passing over a membrane.

It is still another object of the invention to provide a method and system utilizing double flows for simultaneously culturing cells suspended in a flow of culture fluid extending across one surface of a membrane and removing waste products from the cell-containing fluids through the membrane from the opposite surface thereof.

A further object of the invention is to provide a method and system for culturing cells suspended in a flow of culture fluid extending across one side of a membrane by supplying nutrients through the membrane by osmotic diffusion.

An additional object of the invention is to provide a method and system for utilizing double flows for

-5-

simultaneously culturing, aerating and otherwise treating cells suspended in a flow of culture fluid extending across one side of a membrane.

It is also an object of the invention to provide a method and system for aerating cells suspended in a flow of culture fluid extending across one side of a membrane by supplying gas to the fluid through the membrane by diffusion.

## BRIEF DESCRIPTION OF THE DRAWINGS

FIG. 1 is a vertical section view of a double flow system for culturing and treating cells suspended in culture fluid flowing across a membrane.

FIG. 2 is a vertical section view of a double flow system for culturing and aerating cells suspended in flowing culture fluid.

FIG. 3 is a vertical section view of a plurality of membranes for a double flow system for culturing and treating cells suspended in a flowing culture fluid.

FIG. 4 is a schematic view of a dual flow system for culturing and providing nutrients to cells suspended in flowing culture fluid.

FIG. 5 is a schematic view of a dual flow system for culturing and aerating cells suspended in flowing culture fluid.

FIG. 6 is a schematic view of a dual flow system for both culturing and aerating cells suspended in flowing culture fluid.

FIG. 7 is a schematic view of a dual flow system for providing nutrients to and removing wastes from cells suspended in flowing culture fluid.

FIG. 8 is a schematic view of an array of dual flow systems for culturing and treating cells suspended in flowing culture fluid.

FIG. 9 is a schematic view of an array of dual flow systems for culturing, providing nutrients to, and aerating cells suspended in flowing culture fluid.

FIG. 10 is a fragmentory perspective view of a multi-flow system for culturing and treating cells suspended in flowing culture fluid.

FIG. 11 is a perspective view of a rigid screen-like support for a membrane.

FIG. 12 is a perspective view of a rigid plate-like support for a membrane.

FIG. 13 is a section view of a solitary-multiflow system.

FIG. 14 is a section view of a cartridge-multiflow system.

FIG. 15 is a vertical section view of a double flow system for culturing and treating cells attached to a membrane.

FIG. 16 is a vertical section view of a double flow system for culturing and aerating cells attached to particles or encapsulated in beads and suspended in flowing culture medium.

## DETAILED DESCRIPTION OF THE INVENTION

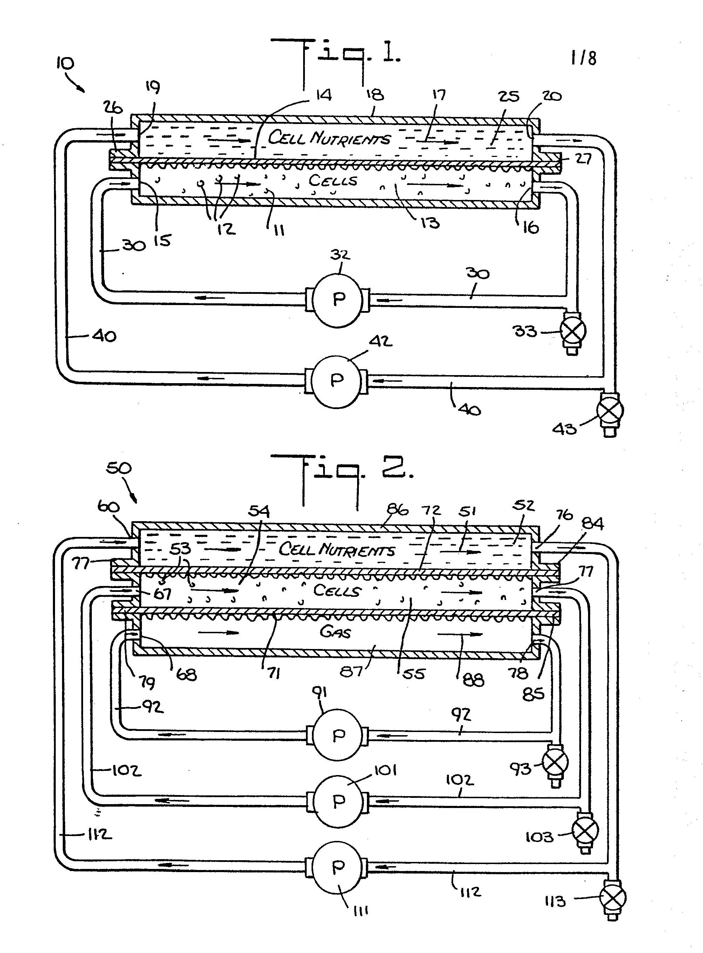

With reference to FIG. 1, the dual flow method and system 10 of the invention is effected by initiating a flow of fluid 11 containing cells 12 through a first compartment 13, and a flow of gas or a liquid 17 containing cell nutrients through a second compartment 25. A membrane 14, preferably a semi-permeable membrane 14, forms a wall common to compartments 13 and 25. The compartments are disposed in housing 18 and are joined at mating areas 26 and 27.

Fluid 11, such as culture fluid, containing cells 12 is introduced into or released system 10 by means of valve 33. The fluid is circulated through compartment 13 by pump 32 and tubing 30. The flow of cell-containing fluid 11 is caused to move continuous with membrane 14 by passing the fluid 11 into compartment 13 through inlet ports 15 and ultimately out of port 16.

It is noted that the fluid 11 being flowed through first compartment 13 is a cell culture fluid which cultures cells 12, or cells contained within beads, or cells attached to particles flowing in the fluid, or cells attached to the surface of membrane 14 within the cell culture fluid compartment 13. Cells may therefore be disposed within the culture fluid of any of the systems, apparati, or methods described herein as freely flowing cells, as cells contained within beads, as cells attached to particles or as cells attached to the surface of a membrane. Such emboidments are more fully described and depicted hereinbelow with reference to FIGS. 15 and 16.

Referring again to FIG. 1 a fluid 17 comprising either a liquid containing cell nutrients or gas such as air or oxygen for aerating a cell is introduced into system 10

-8-

by port 43 and is circulated through compartment 25 by pump 42 and tubing 40. The flow of a fluid 17 containing cell nutrients or gas 17 is directed to move contiguous to membrane 14 by causing the fluid or gas 17 to enter compartment 25 through inlet port 19 and to exit through outlet port 20.

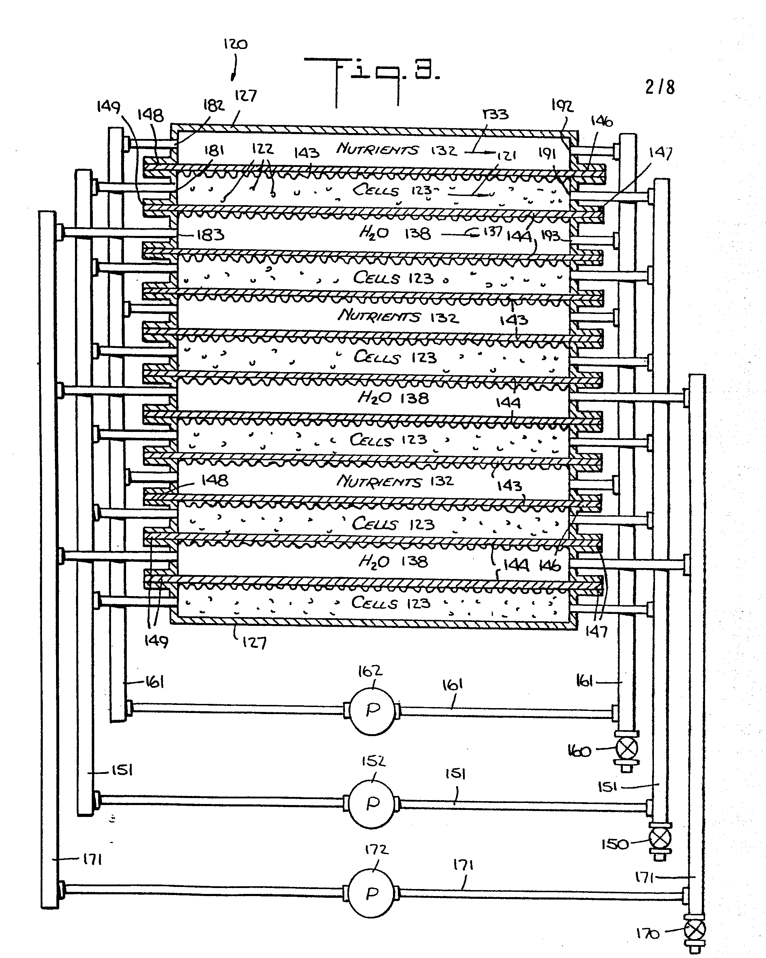

With reference to the embodiment of FIG. 2, the double flow method and system of the invention is effected by initiating a flow of fluid 54 containing cells 53 through a first compartment 55, and a flow of fluid containing cell nutrients 51 through a second compartment 52, and a flow of gas or fluid free from cell waste 88 through a third compartment 87. A first semi-permeable membrane 72 and a second semi-permeable membrane 71 are surrounded and supported by housing 86 at mating areas 77, 79, 84, 85 to form compartments 52, 55, 87 respectively. Fluid 53 containing cells 54 is introduced into or released from system 50 by means of valve 103. Fluid 53 such as culture fluid is circulated through compartment 55 by pump 101 and tubing 102. The flow of cell containing fluid 53 is directed to move contiguous with respect to each of membranes 72 and 71 by the introduction of the fluid 53 into inlet port 67 and by the subsequent release of culture fluid from outlet port 77.

Cell nutrient containing fluid 51 is introduced into or released from system 50 of FIG. 2 valve 113 and circulated through compartment 52 by pump 111 and tubing 112. The flow of cell nutrient containing fluid 51 is established to be contiguous to contact membrane 72 by introduction of fluid 51 into inlet port 60 of compartment 52 and by release through outlet port 76.

Gas or fluid free from waste 88 is introduced into or vented out of system 50 of FIG. 2 by port 93 and circulated through compartment 87 by pump 91 and tubing 92. The flow of gas or fluid 88 is directed to be continguous with membrane 71 by introduction of gas or fluid 88 into and out of compartment 87 through inlet port 68 and outlet port 78.

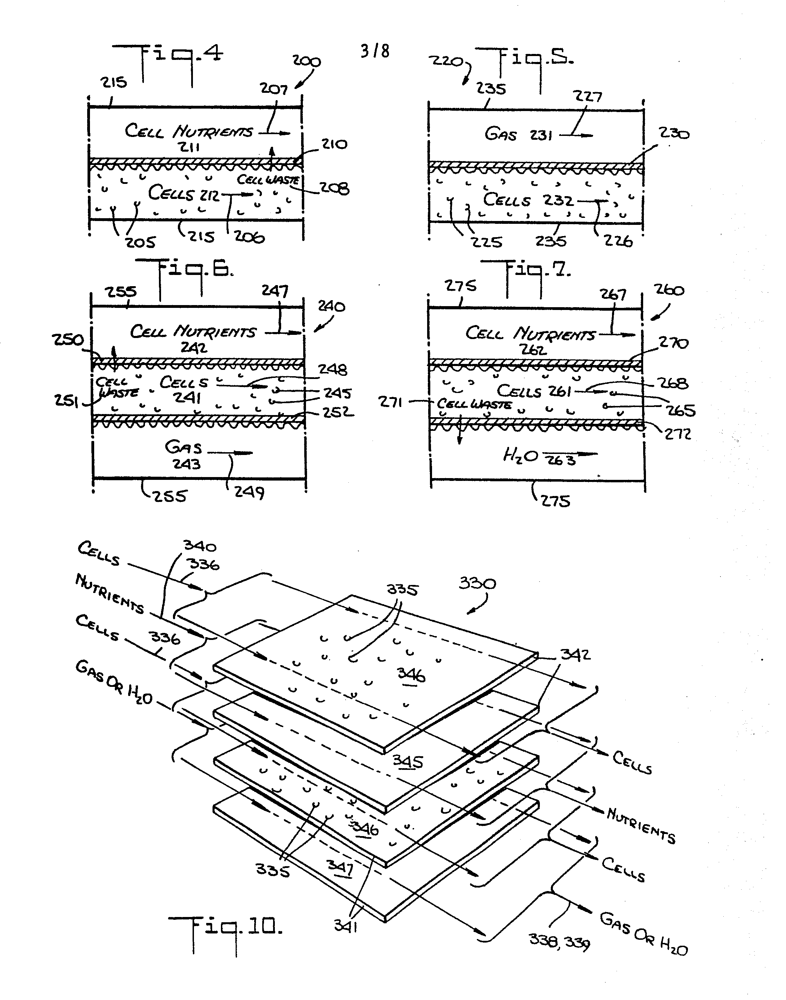

With reference to FIG. 3, the multiflow method and system 120 of the present invention is effected by initiating a flow of fluid 121 containing cells 122 through a plurality of first compartments 123, and initiating a flow of cell nutrients 133 through a plurality of second compartments 132, and initiating a flow of gas or fluid free from cell waste 137 through a plurality of third compartments 138. A plurality of pairs of first semi-permeable membranes 143 and a plurality of pairs of second semi-permeable membranes 144 are arranged in alternating sequence within housing 127. Membranes 143, 144 mate with housing 127 at mating areas 148, 149, 146, 147 to form a plurality of first, second and third compartments 123, 132, 138.

In FIG. 3 fluid 121 containing cells 122 is introduced into or vented out of system 120 by valve 150 and circulated through compartments 123 by pump 152 and tubing 151. The flow of cell containing fluid 121 is maintained parallel to and in contact with either or both a first semi-permeable membrane 144 by introduction of the fluid 122, 121 into and out of the plurality of compartments 123 through inlet and outlet ports 181, 191.

In FIG. 3, cell nutrient containing fluid 133 is introduced into or vented out of system 120 by valve 160 and circulated through compartments 132 by pump 162 and tubing 161. The flow of cell nutrient containing fluid 133 is passed parallel to and in contact with a pair of first semi-permeable membranes 143 by introducing cell nutrient containing fluid into and out of compartments 132 through inlet and outlet ports 181, 182.

In FIG. 3 gas or fluid free from waste 137 is introduced into or vented out of system 120 via port 170 and circulated through compartments 138 by pump 172 and tubing 171. The flow of gas or fluid 137 is directed parallel to and in contact with a pair of second semi-permeable membranes 144 by introducing gas or fluid 137 into and out of compartments 138 through inlet and outlet ports 183, 193.

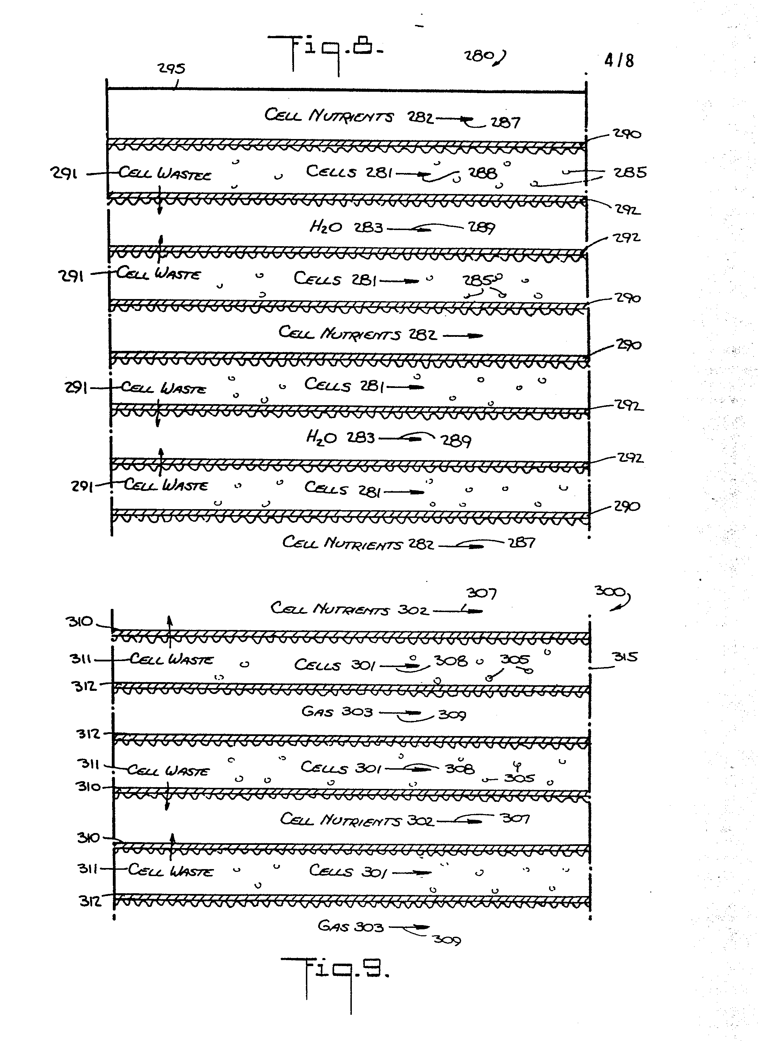

With reference to FIG. 4, one embodiment of the invention provides a dual flow system 200 for simultaneously culturing and treating cells 205 suspended in a culture fluid 206. The system comprises a semi-permeable membrane 210 which is permeable to selected cell nutrients 207 and selected cell waste products 208. A housing 215 surrounds and supports the membrane 210 to form two separate compartment 211, 212. Compartment 211 is above while compartments 212 is below the membrane 210. An inlet port leading into each compartment 211, 212 and an outlet port leading from each compartment 211, 212. The inlet and outlet ports are positioned within each compartment 211, 212 to enable a cell containing fluid 205, 206 to be flowed through one of the compartments 212 parallel to and in contact with the membrane 210. A fluid which contains the selected cell nutrients 207 and which is free from selected waste products 208 is flowed through the other of the compartments 211 parallel to and in contact with the membrane.

Also with reference to FIG. 4, the dual flow method of culturing and treating cells comprises maintaining a flow of the cell containing fluid 205, 206 through a first compartment 212 parallel to and in contact with a wall 210 of the first compartment 212. The wall 210 is a semi-permeable membrane 210 which is permeable to selected cell nutrients 207 and selected cell waste products 208. The method further comprises passing a fluid which contains selected cell nutrients 207 and which is free from selected cell waste products 208 through a second compartment 211, parallel to and in contact with a wall 210 of the second leakproof compartment 211. The wall 210 of the second leakproof compartment 211 is the same semi-permeable membrane 210 which comprises the wall 210 of the first leakproof compartment 212. The cells 205 are cultured during flow by selective diffusion of cell nutrients through membrane 210 into fluid 206.

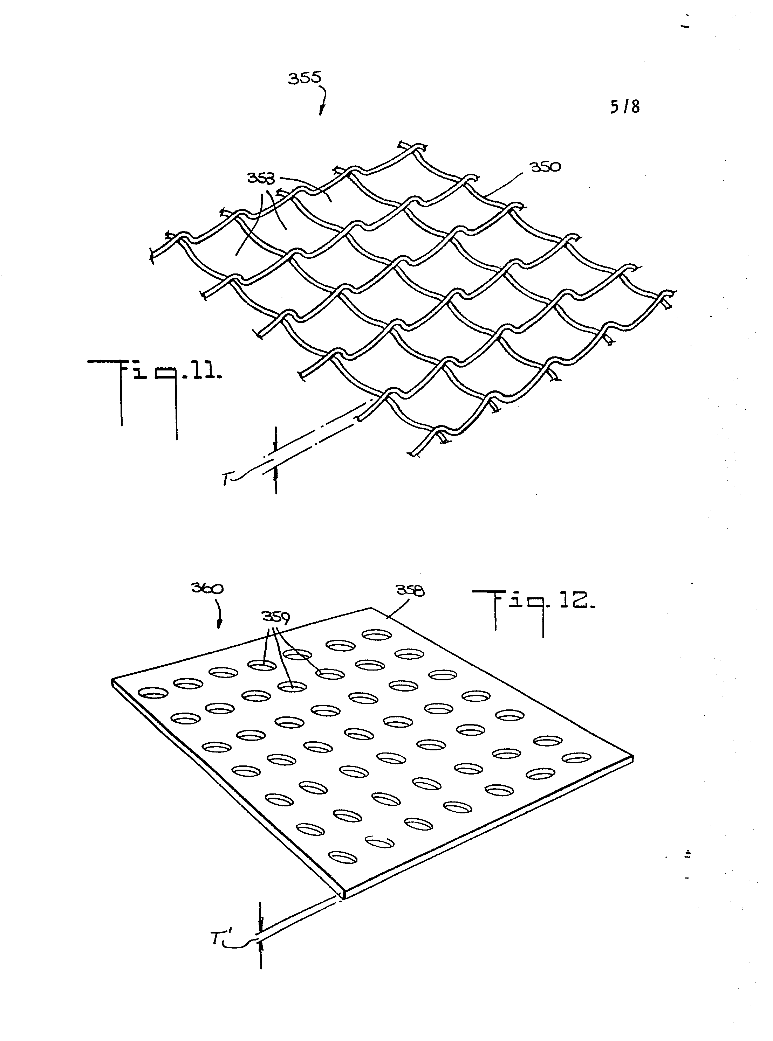

With reference to FIG. 5, another embodiment of the invention provides a dual flow aerating system 220 comprising a semi-permeable membrane 230 which is permeable to selected gases 227 and is impermeable to solids and fluids. A housing 235 which surrounds and supports the membrane 230 forms separate compartments 231, 232 with one of the compartments 231 being above and the other 232 below the membrane 230. An inlet port leads into each compartment 231, 232 and an outlet port leads out of each compartment 231, 232. The inlet and outlet ports are positioned within each compartment 231, 232 to enable a cell containing fluid 225, 226 to be flowed through one of the compartments 232, parallel to and in contact with the membrane 230. Selected gases 227 are flowed through the other of the compartments 231, parallel to and in contact with the membrane 230, and aerate cells 225 by selective diffusion through membrane 230 and into fluid 226.

Also with reference to FIG. 5, a dual flow method of aerating the culture fluid 225, 226 is provided comprising maintaining a flow of the cell containing fluid 225, 226 through the first compartment 232 parallel to and in contact with a wall 230 of the first compartment 232. The wall 230 is a semi-permeable membrane 230 which is permeable to selected gases 227 and impermeable to solids and liquids. The method further comprises passing the selected gases 227 through a second leakproof compartment 231, parallel to and in contact with a wall 230 of the second leakproof compartment 231. The wall 230 comprises the semi-permeable membrane 230 which also comprises a wall 230 of the first leakproof compartment 232.

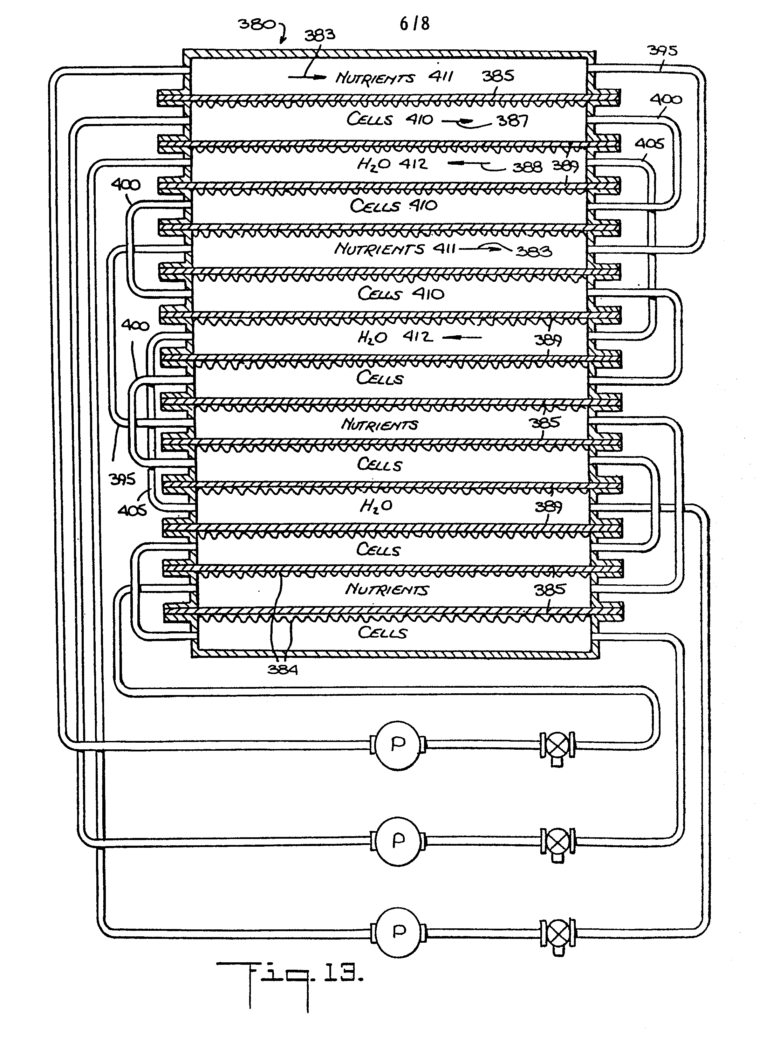

With reference to FIG. 6, another embodiment of the invention provides a double flow filter system 240 comprising a first semi-permeable membrane 250 which is permeable to selected cell nutrients 247 and selected cell waste products 251, and a second semi-permeable membrane 252 which is permeable to selected gases 249 and impermeable to solids and fluids. The system includes a housing 255 which surrounds and supports the membranes 250, 252 in parallel and spaced relationship with respect to one another whereby different compartments are formed above 242 the first membrane 250, below 243 the second membrane 252, and between 241 the two membranes 250, 252. An inlet port leads into each compartment and an outlet port leads out of each compartment 241, 242, 243. The inlet and outlet ports are positioned within each compartment 241, 242, 243 to enable a cell containing fluid 245, 248 to flow through one compartment 241 between, parallel to, and in contact with the two membranes 250, 252. The ports also enable a fluid which contains the selected cell nutrients 247 and is free from the selected cell waste products 251 to flow through another compartment 242 , parallel to and in contact with the first membrane 250. The ports also enable the selected gases 249 to flow through another compartment 243 parallel to and in contact with the second membrane 252. Simultaneous culturing, filtering and aerating is achieved by selective diffusion of nutrients 247 through membrane 250 into fluid 248, selective diffusion of cell waste 251 through membrane 250 into compartment 242 and selective diffusion of gas through membrane 250 into fluid 248.

Also with reference to FIG. 6, a double flow method of simultaneously culturing, filtering and aerating culture fluid 245, 248 is provided comprising maintaining a flow of the culture fluid 245, 248 through a first compartment 241, parallel to and in contact with the walls 250, 252 of the first compartment 241, the walls comprising a first semi-permeable membrane 250 which is permeable to the selected cell nutrients 247 and the selected cell waste products 251, and a second semi-permeable membrane 252 which is permeable to the selected gases 249 and impermeable to solids and fluids. The method further comprises passing a fluid which contains the selected cell nutrients 247 and is free from the selected cell waste products 251 through a second compartment 242, parallel to and in contact with a wall 250 of the second compartment 242. The wall of the second compartment comprises the same first semi-permeable membrane 250 which also comprises a wall 250 of the first compartment 241. The method additionally includes directing the selected gases 249 through a third compartment 243 parallel to and in contact with a wall 252 of the third compartment 243, the wall of the third leakproof compartment 243 comprising the second semi-permeable membrane 252 which also comprises a wall ∃ of the first compartment 241.

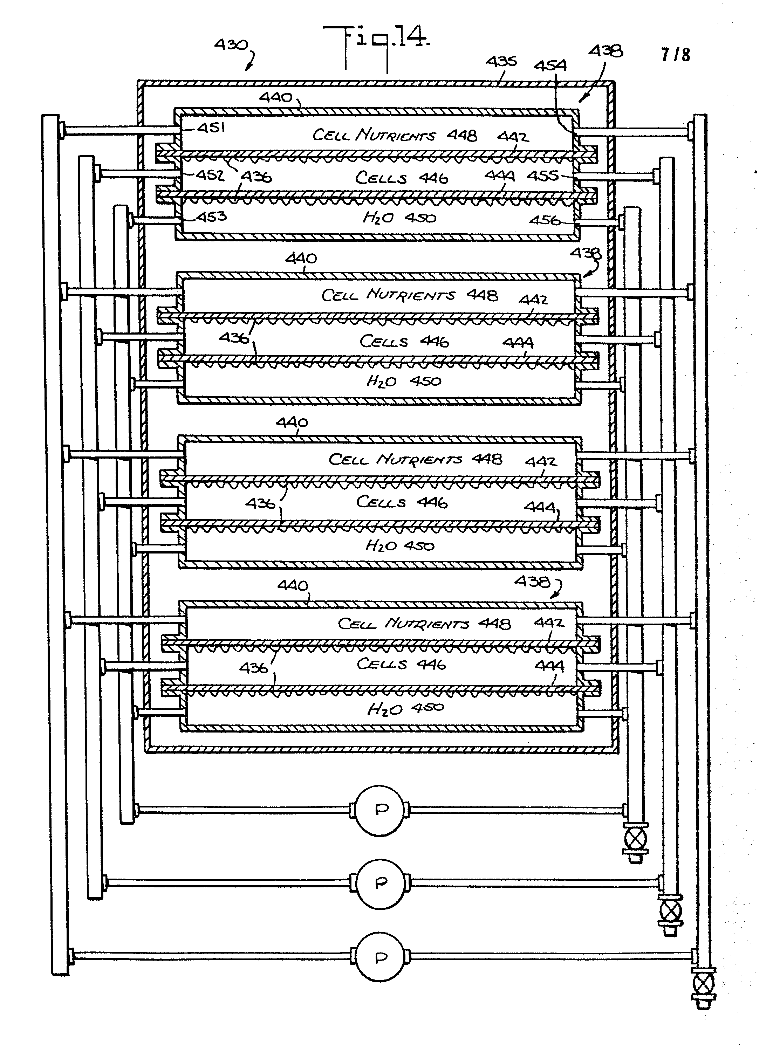

With reference to FIG. 7, another embodiment of the invention provides a double flow filter system 260 comprising a first semi-permeable membrane 270 which is permeable to selected cell nutrients 267 and impermeable to selected cell waste products 271, a second semi-permeable membrane 272 which is permeable to the selected cell waste products 271, a housing 275 which surrounds and supports the membranes 270, 272 in a parallel and spaced relationship with respect to one another whereby a different compartment is formed above 262 the first membrane 270, below 263 the second membrane 272, and between 261 the two membranes 270, 272, an inlet port leading into each compartment 261, 262, 263 and an outlet leading out of each compartment. The inlet ports and the outlet ports are positioned within each compartment 261, 262, 263 to respectively enable a cell containing fluid 265, 268 to flow through one compartment 261 between, parallel to and in contact with the two membranes 270, 272, to enable a fluid containing the selected cell nutrients 267 to flow through another compartment 262 parallel to and in contact with the first membrane 270, and to enable a fluid which is free from the selected cell waste products 269 to flow through another compartment 263 parallel to and in contact with the second membrane 272. Simultaneous culturing and filtering of cells 265 is achieved by selective diffusion of cell waste 271 through membrane 272 into fluid 269 and selective diffusion of cell nutrients 267 through membrane 270 into fluid 268.

Also with reference to FIG. 7, a double flow method of simultaneously culturing and filtering such culture fluids is provided comprising maintaining a flow of the culture fluid 265, 268 through a first compartment 261, parallel to and in contact with the walls 270, 272 of the first compartment 261 in which the walls 270, 272 comprise a first semi-permeable membrane 270 which is permeable to selected cell nutrients 267 and impermeable to selected cell waste products 271 and a second semi-permeable membrane 171 which is permeable to the

-15-

selected cell waste products 271. The method also comprises passing a fluid containing the selected cell nutrients 267 through a second compartment 262, parallel to and in contact with a wall 270 of the second compartment 262 in which the wall 270 of the second compartment 262 comprises the first semi-permeable membrane 270 which also comprises a wall 270 of the first compartment 261. The method also includes directing a fluid 269 which is free from the selected cell waste products 271 through a third compartment 263, parallel to and in contact with a wall 272 of the third compartment 263 such that the wall 272 comprises the second semi-permeable membrane 272 which also comprises a wall 272 of the first compartment 261.

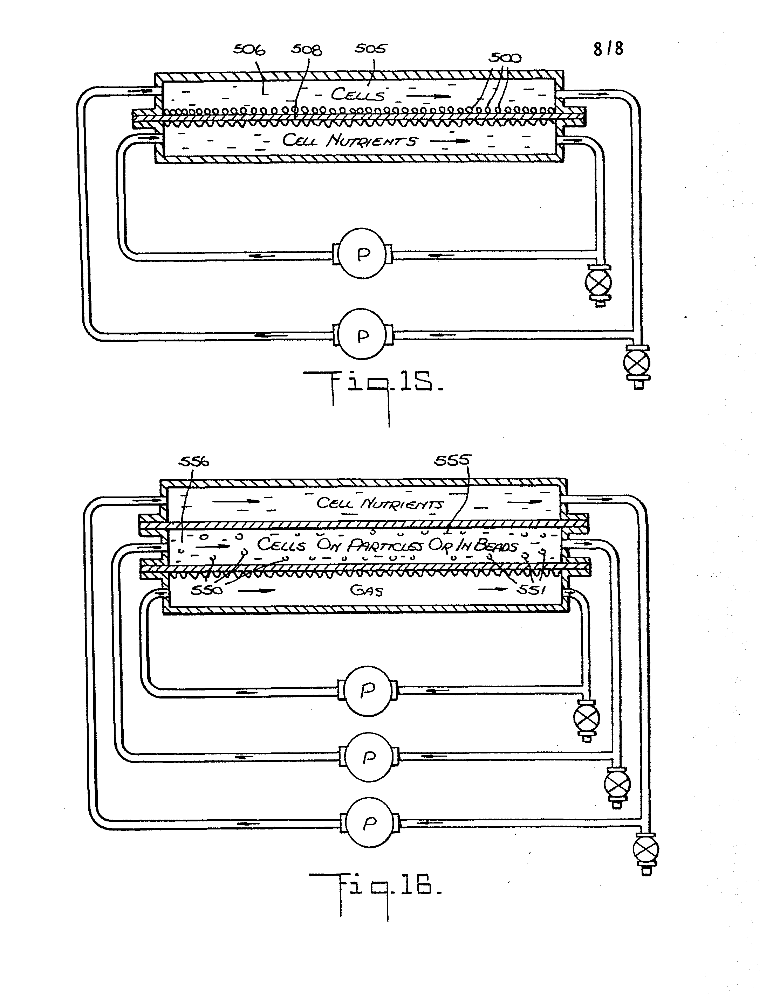

With reference to FIG. 8, another embodiment of the invention provides a multi-flow filter system 280 comprising a plurality of pairs of first semi-permeable membranes 290 which are permeable to selected cell nutrients 287 and impermeable to selected cell waste products 291, a plurality of pairs of second semi-permeable membranes 292 which are permeable to the selected cell waste products 291, a housing 295 which surrounds and supports the plurality of pairs of first 290 and second membranes 292 such that the pairs are arranged in alternating sequence within the housing 295 and each individual membrane 290, 292 is disposed in a parallel and spaced relationship with respect to an adjacent membrane to form a series of separate compartments 281, 282, 283 between the membranes 290, 292, an inlet port leading into each compartment 281, 282, 283, and an outlet port leading out of each compartment such that the inlet and outlet ports are positioned within each compartment to respectively enable a cell containing fluid 285, 288 to flow through one compartment 281 between, parallel to and in contact with adjacent first 290 and second 292 membranes, and to enable a fluid containing the selected cell nutrients 287 to flow through another compartment 282 between, parallel to and in contact with a pair of first semi-permeable membranes 290, and to enable a fluid 289 which is free from the selected cell waste products 291 to flow through another compartment 282 between, parallel to and in contact with a pair of the second semi-permeable membranes 292. Simultaneous culturing and filtering of cells 285 is achieved by selective diffusion of cell nutrients 287 through membranes 290 into fluid 288 and selective diffusion of cell waste products 291 through membranes 292 into fluid 289.

Also with reference to FIG. 8, a multi-flow means of simultaneously culturing and filtering such culture fluids is provided comprising maintaining a flow of the culture fluid 285, 288 through a plurality of first compartments 281, parallel to and in contact with the walls 290, 292 of the first compartment 281 such that the walls of each of the first compartments 281 comprise a first semi-permeable membrane 290 which is permeable to selected cell nutrients 287 and impermeable to selected cell waste products 291 and, a second semi-permeable membrane 292 which is permeable to the selected cell waste products 291. This method further comprises passing a fluid which contains the selected cell nutrients 287 through a plurality of second compartments 282, parallel to and in contact with the walls 290 of the second compartments 282 such that the walls 290 comprise a pair of the first semi-permeable membranes 290 which also comprise one of the walls 290 of each of a spaced pair of the first compartments 281; and the method includes directing a fluid 289 free from the selected cell waste products 291 through a plurality of third compartments 283, parallel to and in contact with the walls 292 of the third compartments 283 such that the walls 292 comprise a pair of the second semi-permeable membranes 292 which also comprise one of the walls 292 of each of a spaced pair of the first compartments 281.

With reference to FIG. 9, another embodiment of the invention provides a multi-flow system 300 comprising a plurality of pairs of first semi-permeable membranes 310 which are permeable to selected cell nutrients 307 and selected cell waste products 311, a plurality of pairs of second permeable membranes 312 which are permeable to selected gases 309 and impermeable to solids and fluids, a housing 315 which surrounds and supports the plurality of pairs of first 310 and second 312 membranes such that the pairs are arranged in alternating sequence within the housing 315 and each individual membrane 310, 312 is disposed in a parallel and spaced relationship with respect to an adjacent membrane to form a series of separate compartments 301, 302, 303 between the membranes, an inlet port leading into each compartment, and an outlet port leading out of each compartment 301, 302, 303 whereby the inlet and outlet ports are positioned within each compartment to enable a cell containing fluid 305, 308 to flow through one compartment 301, between, parallel to and in contact with adjacent first 310 and second 312 membranes, and to enable a fluid which contains the selected cell nutrients 307 and is free from the selected cell waste products 311 to flow through another compartment 302 between, parallel to and in contact with a pair of first membranes 310, and to enable the selected gases 309 to flow through another compartment 303 between, parallel to and in contact with a pair of second membranes 312. Simultaneous culturing, filtering and aeration of cells 305 is achieved by selective diffusion of nutrients 307 through membranes 310 into fluid 308, selective diffusion of cell waste 311 through membranes 310 into compartment 302 and selective diffusion of gas 309

-18-

through membranes 312 into fluid 308.

Also with reference to FIG. 9, a multi-flow means of simultaneosuly aerating, culturing and filtering such culture fluids 305, 308 is provided comprising maintaining a flow of the culture fluid 305, 308 through a plurality of first compartments 301, parallel to and in contact with the walls 310, 312 of the first compartments 301 such that the walls of the first compartments comprise a first semi-permeable membrane 310 which is permeable to selected cell nutrients 307 and selected cell waste products 311, and a second semi-permeable membrane 312 which is permeable to selected gases 309 and impermeable to solids and liquids. This method further comprises passing a fluid which contains the selected cell nutrients 307 and is free from the selected cell waste products 311 through a plurality of second compartments 302, parallel to and in contact with the walls 310 of the second compartments 302 such that the walls 310 comprise a pair of the first semi-permeable membranes which also comprise one of the walls 310 of each of a spaced pair of the first compartments 301; and the method includes directing selected gases 309 through a plurality of third compartments 303, parallel to and in contact with the walls 312 of the third compartments 303 such that the walls 312 comprise a pair of the second semi-permeable membranes 312 which also comprise one of the walls 312 of each of a spaced pair of the first compartments 301.

FIG. 10 depicts the embodiments of FIGS. 9 and 10 in a transverse section view, showing a generalized multiflow system 340 having a plurality of first compartments 346 formed by the spacing apart of first semi-permeable membranes 342 and second semi-permeable membranes 341 in parallel relationship to each other. A plurality of second compartments 345 are formed by the spacing apart of pairs of first semi-permeable membranes 342 in parallel relationship to each other and a plurality of third compartments 347 are formed by the parallel spacing apart of pairs of second semi-permeable membranes 341.

It can be seen from FIG. 10 that a fluid 336 containing cells 335 is flowed through the first compartments 346 parallel to and in contact with the membranes 341, 342 which comprise either of the walls of the first compartments 346. In the multiflow system 330, a cell nutrient containing fluid 340 is flowed through second compartments 345 having first semi-permeable membranes 342 in common with adjacent first compartments 346, parallel to and in contact with a pair of first semi-permeable membranes 342. Finally in the multiflow system 330, either a fluid free from selected cell waste products 338 or selected gases 339 are flowed through third compartments 347 having second semi-permeable membranes 341 in common with first compartments 346, parallel to and in contact with the second membranes 341.

As can be seen from FIG. 10, the flows of all fluids 340, 338, 336 and/or gases 339 are being flowed in the same horizontal direction while parallel to each other in the vertical direction. It should be noted however that the horizontal directions of the flows of the various fluids 340, 338, 336 and gases 339 as indicated by the arrows of FIG. 10 are exemplary only, and that the direction of flow through each individual compartment may be maintained in any horizontal direction, from 0-360 degrees, relative to the horizontal direction in any other compartment, the only spatial limitation on the direction of flow in any given compartment being that it must be maintained parallel to the planar surfaces of vertically parallel, spaced membranes 341, 342 which form the walls of a given compartment.

The choice of a specific membrane which is suitable for use in the present invention depends upon the user's choices as to specific cell nutrients to be used in culturing the cells, specific cell waste products desired to be filtered, and specific gases selected for aeration of a given cell culture fluid.

Inasmuch as there are basically two microstructural forms of membranes, porous, non-porous and something in between the two, any of which may possess a chemical structure ranging from a simple hydrocarbon to polar or ionic structures, the user has a wide variety of chemical structures to choose from depending upon the chemical species intended to be diffused through a given membrane from one fluid flowing through one compartment in relatively high concentration to another fluid flowing in an adjacent compartment in relatively low concentration. Most preferably the membranes chosen for use in the present invention allow for a relatively rapid rate of diffusion and are highly specific for diffusion of the materials selected for culturing, filtering, or aeration.

Examples of polymers for potential use as membranes are cellulose, polyethylene and polypropylene which are non-polar, polyamides which are polar, and olefin polymers which are ionic and in which cations or anions are attached to the backbone of the polymer. Some examples of microporous membrane materials which are of potential use in the present invention are cellulose esters, polyvinylchloride, certain aromatic polymers, polytetrafluoroethylene, polypropylene, polycarbonate, polystyrene, polyethylene, and nylon. In the case where selective diffusion of gas is intended, a rubber

-21-

material would preferably be employed.

Most preferably the present invention contemplates the absence of any substantial pressure differential between adjacent compartments, which systems would obviously require the pressures of each separately flowing stream in all compartments to be equal. In those instances, however, where maintenance of substantially equal pressures in separate compartments is impracticable, the membrane(s) which forms the wall or barrier between adjacent compartments in which the pressures may be unequal, may be attached to a rigid support having openings to allow the membrane to contact fluids flowing on opposite surfaces thereof and thereby still allow normal osmotic diffusion to occur through the membranes.

FIGS. 11 and 12 depict in general form two embodiments of rigid supports contemplated for use in supporting any membrane to be employed in any of the systems or apparati discussed herein.

FIG. 11 depicts one kind of support 355 which is made up of a series of relatively rigid supports 350 which are inert to the fluids intended for use in the present invention and are intertwined with each other to form a generally planar and rigid screen 355 onto which a membrane could be permanently mounted. Screen 355 would obviously possess a plurality of holes or perforations 353 very gross in size relative to molecular dimensions through which the various fluids contemplated for use in the present invention would be enabled to contact one surface of the membrane mounted on the screen 355 thereby allowing normal osmotic diffusion to occur through the membrane. The width T of the screen 355 is deliberately kept to a relatively small dimension, i.e. less than several millimeters in order to prevent pockets of fluid or diffusing materials from becoming statically entrenched within perforations 353 and to enable such substances which enter perforations 353 to be readily flushed into the flow of fluid which is passed tangentially along the generally planar surface of screen 355.

FIG. 12 depicts another sort of support 360 for a membrane. The support 360 is constructed of a relatively rigid plate 358 which is inert to the fluids and materials intended to be flowed across the plate 360 and membrane, and contains a plurality of holes or perforations 359 through which a flowing fluid may contact a membrane mounted on the plate 358. As with the screen 355 of FIG. 11, the thickness T' of plate 360 is kept to a relatively small dimension, i.e. less than several millimeters.

Fig. 13 depicts a varied embodiment 380 of the multiflow systems of Figs. 3, 8 and 9 wherein the three fluids depicted as flowing through the system of FIG. 13 are cell containing fluid 387, cell nutrient containing fluid 383, and fluid free from cell waste 388. Each fluid is passed through a plurality of compartments in solitary flows as opposed to separate flows as depicted in Figs. 3, 8 and 9.

The solitary flows of each fluid 387, 383 and 388 are maintained by connecting the outflows of each of cell containing fluid's compartment 410 to the inflows of its successive compartments 410 via tubing 400. Similarly the outflows of cell nutrient containing fluid's compartments 411 are connected to the inflows of successive compartments 411 by tubing 395, and the outflows of compartments 412 through which fluid free from waste 388 is passing are connected to the inflows of compartments 412 by tubing 405. As in the multiflow systems of Figs. 3, 8 and 9, the system 380 of Fig. 13 possesses a plurality of compartments 410, 411, 412, and the walls of each compartment 410 are comprised of a first semi-permeable 385 and a second semi-permeable membrane 389. Whereas the fluids chosen for depiction in FIG. 13 are a cell nutrient containing fluid 383 and a fluid free from cell waste 388, the first semi-permeable membrane 385 is selectively permeable to the selected cell nutrients and the second semi-permeable membrane 389 is selectively permeable to selected cell waste products. Also depicted in the system 380 of Fig. 13 are rigid supports 384 for each of the first and second semi-permeable membranes 385, 389 in order to prevent the membranes 385, 389 from rupturing due to pressure differentials which might exist between compartments 410, 411, 412.

Fig. 14 depicts another embodiment of a multiflow system 430 which is comprised of a series of cartridges 438 arranged sequentially within a master housing 435. Each cartridge 438 is comprised of a first semi-permeable membrane 442, a second semi-permeable membrane 444, and a housing 440 which surrounds and supports membranes 442, 444 in parallel spaced relationship to each other. Each membrane 442, 444 is supported against rupture due to potential pressure differences between compartments 446, 448, 450 by rigid supports 436.

Each cartridge 438 as depicted in Fig. 14 comprises a separate triple flow system similar to the system described and depicted with reference to Figs. 2 and 7, wherein the first semi-permeable membrane 442 is permeable to selected cell nutrients and impermeable to selected cell waste products, and the second semi-permeable membrane 444 is permeable to the selected cell waste products. Also similar to the triple flow system of Figs. 2 and 7, each cartridge 438 has three separate compartments 446, 448 and 450 through which respectively

-24-

are flowed a cell containing fluid, a cell nutrient containing fluid and a fluid free from the selected cell waste products; and each compartment 446, 448 and 450 of each cartridge 438 respectively possesses inlet ports 452, 451, 453 and outlet ports 455, 454, 456 which are strategically positioned about the housings 440 of each cartridge 438 to enable the flows of all fluids to pass parallel to and in contact the surfaces of membranes 442, 444.

In addition to the specific triple flow embodiments of the cartridges 438 described with reference to Fig. 4, other embodiments of the cartridge system of Fig. 4 can be imagined wherein each cartridge embodies a dual flow system as described and depicted with reference to Figs. 1, 4, and 5, or each cartridge could embody a multiflow system as described and depicted with reference to Figs. 3, 8 and 9.

The purpose of providing a cartridge system as described with reference to Fig. 14, is to allow easy replacement of an individual cartridge 438 in the event one or more of membranes 442, 444 should degrade or rupture, while at the same time maintaining the culture system 430 operative as a whole, and to allow conversion of the system 430 to one including dual or multiflow cartridges or cartridges having different semi-permeable membranes which are selectively permeable to other and different materials from those materials originally selected for diffusion.

With reference to FIG. 15 the double flow system of FIG. 1 is shown wherein the cells 500 to be cultured by culture fluid 506 are attached in stationary positions, relative to the flow of culture fluid 506, to the semi-permeable membrane 508 within the culture fluid compartment 505.

With reference to FIG. 16 the double flow system of FIG. 3 is depicted wherein the cells to be cultured by culture fluid 555 are either attached to particles 550 or contained within beads 551 which are flowing freely with culture fluid 555 within culture fluid compartment 556.

As can be readily imagined such membrane attached, particle attached or bead encapsulated cells as described with reference to FIGS. 15 and 16 may be employed in lieu of freely flowing cells in any of the systems, apparati, and methods described herein.

Inasmuch as the macroscopic structure of the membranes contemplated for use in the present invention are that of a relatively thin sheet, a relatively rigid support plate may be necessary to prevent the rupturing of the membrane in those cases where the pressure differential between adjacent compartments might be sufficient to cause the membrane to rupture. The present invention preferably contemplates, the filtering of material by diffusion alone, through a membrane and into a flowing stream, whereby the filtrate is carried out of the system in the fluid into which it has diffused, thereby rendering the need for cleaning of the filter unnecessary. The preference for employment of equal pressures in each compartment of the filter systems described herein is highlighted by the fact that an ideal osmotic diffusion may be achieved which is dependent only upon concentration differences, in contrast to a system where pressure differences across a given membrane also create a further variable in the rate and kind of diffusion which takes place through certain membranes.

It is also noted that in those cases in the systems, apparati and methods described herein where a pressure differential between adjacent compartments may exist, filtration will occur whereby a mass flow of water and other materials may take place through the membrane. The pressure differential will therefore effect a mass flow of materials, in addition to the flow of materials through a membane by virtue of the difference in concentration of the materials dissolved in the fluids flowing on either side of the membrane.

CLAIMS

1.      A method utilizing dual flow for culturing substances such as mammalian cells, parasitic organisms, and the like when disposed directly in a fluid culture medium characterised by the steps of:
delivering a flow of the fluid culture medium in which the substances to be cultured are disposed to one surface of a membrane, the flow of the culture fluid extending across the one surface contiguous therewith, the membrane having exposed oppositely disposed surfaces and being permeable to at least one of different materials including nutrients for the substances, waste products from the substances, and gas for treating the substances, and
directing a flow of at least one of the different materials to the other of the oppositely disposed surfaces of the membrane, the flow of the material extending across the other surface contiguous therewith, the dual flow of the substances to be cultured and at least one of the different materials with respect to the membrane enabling at least one of the different materials to diffuse with respect to the membrane during culturing of the substances.

2.      A method utilizing dual flow for culturing substances in accordance with claim 1 characterised in that the steps of delivering a flow of the fluid culture medium and the directing of a flow of at least one of the different materials comprise flows which are in at least one of the same direction, opposite directions, and transverse cross-flow directions.

3.         A method utilizing dual flow for culturing substances in accordance with claim 1 or 2 and characterised by the step of recirculating the fluid culture medium for sequential flow across the one surface of the membrane.

4.         A method utilizing dual flow for culturing substances in accordance with any one of the preceding claims and characterised by the step of returning at least one of the different materials for repetitive flow across the other surface of the membrane.

5.         A method utilizing dual flow for culturing substances in accordance with any one of the preceding claims and characterised by the step of attaching the substances to the one surface of the membrane.

6.         A dual flow system for culturing substances such as mammalian cells, parasitic organisms, and the like when disposed directly in a fluid culture medium or attached to particles or encapsulated in beads disposed in a fluid culture medium characterised by:
         a membrane (14) having oppositely disposed surfaces and being permeable to at least one of different materials including nutrients for the substances, waste products from the substances, and gas for treating the substances,
         means (18,26,27) for supporting the membrane with the oppositely disposed surfaces thereof exposed for flow contiguous therewith,
         means (32) for delivering a flow of the fluid culture medium in which the substances to be cultured are disposed to one surface of the membrane, the flow of the culture fluid extending across the one surface contiguous therewith, and

means (42) for directing a flow of at least one of the different materials to the other of the oppositely disposed surfaces of the membrane, the flow of the material extending across the other surface contiguous therewith, the membrane enabling at least one of the different materials when flowing contiguous to the other surface of the membrane to diffuse with respect thereto during culturing of the substances in the flow of the culture fluid extending contiguous with the one surface of the membrane.

7.      A dual flow system for culturing substances in accordance with claim 6 characterised in that the flow of the means (32) for delivering a flow of the fluid culture medium and the flow of the means (42) for directing a flow of at least one of the different materials are related in direction to one another in at least one of the same direction, opposite directions, and transverse cross-flow directions.

8.      A dual flow system for culturing substances in accordance with claim 6 or 7 and characterised by means connected to the means for delivering a flow of the fluid culture medium for recirculating the fluid culture medium for sequential flow across the one surface of the membrane.

9.      A dual flow system for culturing substances in accordance with claim 6,7 or 8 characterised by means connected to the means for directing a flow of at least one of the different materials for returning at least one of the different materials for repetitive flow across the other surface of the membrane.

10. A dual flow system for culturing substances in accordance with any one of claims 6 to 9 and characterised by:

(a) means for mounting a plurality of dual flow systems in an array, and

(b) means for interconnecting the flow delivering means and the flow directing means of each of the plurality of dual flow systems in the array to provide at least one of parallel and series flows of the flow of the fluid culture medium and the flow of at least one of the different materials with respect to each of the plurality of dual flow systems, the membrane of each of the dual flow systems of the array enabling at least one of the different materials to diffuse with respect thereto during culturing of the substances.

11. A dual flow system for culturing substances in accordance with claim 10 characterised in that the means for mounting the plurality of dual flow systems in an array comprises mounting the systems in an array in which the one surface of the membrane to which is delivered the flow of fluid culture medium is in a facing arrangement proximate the one surface of the membrane of the systems adjacent thereto, the facing one surface of the system forming a passage to receive the flow of fluid culture medium thereinto.

12. A dual flow system for culturing substances in accordance with claim 10 or 11 and characterised by a housing surrounding and supporting the membrane, the housing forming two separate compartments with one of the compartments above and the other of the compartments below the membrane, the housing including an inlet port leading into each compartment and an outlet port leading out of each compartment, the inlet and outlet ports being positioned within each compartment to enable a fluid in which the substances are adapted to be treated to flow through one of the compartments parallel to and in contact with the membrane and to enable a fluid containing selected substances to flow through the other of the compartments parallel to and in contact with said membrane.

13.    A dual flow system in accordance with any one of claims 6 to 12 characterised in that the membrane is adapted to have the substances attached thereto.

Fig. 1.

Fig. 2.

Fig. 3.

0112155

2/8

## Fig.4.

Cell Nutrients 211

Cell Waste

Cells 212

## Fig.5.

Gas 231

Cells 232

## Fig.6.

Cell Nutrients 242

Cell Waste

Cells 241

Gas 243

## Fig.7.

Cell Nutrients 262

Cells 261

Cell Waste

$H_2O$ 263

## Fig.10.

Cells
Nutrients
Cells
Gas Or $H_2O$

Cells
Nutrients
Cells
Gas Or $H_2O$

# Fig.8.

280)

295

CELL NUTRIENTS 282 ⟶ 287

~290

291 — CELL WASTE      CELLS 281 ⟶ 288          285

~292

$H_2O$ 283 ⟶ 289

~292

291 — CELL WASTE      CELLS 281 ⟶ 285

~290

CELL NUTRIENTS 282 ⟶

~290

291 — CELL WASTE      CELLS 281 ⟶

~292

$H_2O$ 283 ⟶ 289

~292

291 — CELL WASTE      CELLS 281 ⟶

~290

CELL NUTRIENTS 282 ⟶ 287

300)

307

CELL NUTRIENTS 302 ⟶

310

311 — CELL WASTE      CELLS 301 ⟶ 308    305          315

312

GAS 303 ⟶ 309

312

311 — CELL WASTE      CELLS 301 ⟶ 308    305

310

CELL NUTRIENTS 302 ⟶ 307

310

311 — CELL WASTE      CELLS 301 ⟶

312

GAS 303 ⟶ 309

# Fig.9.

355

353

350

Fig.11.

T

Fig. 12.

360

359

358

T'

Fig. 13.

Fig.14.

430

435 454 438

440

451 CELL NUTRIENTS 448 442

452 436 CELLS 446 444 455

453 H₂O 450 456

440 438

CELL NUTRIENTS 448 442

436 CELLS 446 444

H₂O 450

440

CELL NUTRIENTS 448 442

436 CELLS 446 444

H₂O 450

440 438

CELL NUTRIENTS 448 442

436 CELLS 446 444

H₂O 450

P

P

P

Fig.15.

Fig.16.